# EUROPEAN PATENT APPLICATION

(11) **EP 4 763 104 A1**
(43) Date of publication of application: **24.06.2026**
(21) Application number: 24859738.7
(22) Date of filing: 27.08.2024
(51) Int. Cl.: A61B 17/135

(54) **HEMOSTATIC INSTRUMENT**

(30) Priority: 28.08.2023 JP 2023137825
(71) Applicant: Terumo Kabushiki Kaisha, Tokyo 151-0072 (JP)
(72) Inventor: TACHIZAKI, Yuma, Fujinomiya-shi, Shizuoka 418-0015 (JP); OUCHI, Tatsuya, Fujinomiya-shi, Shizuoka 418-0015 (JP); USAMI, Masashi, Tokyo 163-1450 (JP)
(74) Representative: Casalonga
(86) International application number: PCT/JP2024/030397
(87) International publication number: WO 2025/047706

(57) **Abstract**

To provide a hemostatic device that makes it possible to easily ascertain whether or not a pressing member and band bodies are appropriately placed when used for hemostasis of puncture sites formed in the right hand and the left hand of a patient; A hemostatic device 10 is placeable for both the right hand H1 and the left hand H2 of a patient according to the position of a puncture site p1 of the patient, and includes a cover member 100 configured to cover the puncture site p and a pressing member 200 connected to the cover member and configured to compress the puncture site, wherein the cover member includes a layout display portion 600 showing a placement example of the hemostatic device for the right hand and the left hand of the patient, the pressing member and the layout display portion are positioned on a center line C1 of a first band body 410 included in the cover member, the layout display portion includes a first graphic portion 610 showing a placement example of the hemostatic device for the right hand of the patient and a second graphic portion 620 showing a placement example of the hemostatic device for the left hand of the patient, and the first graphic portion and the second graphic portion are arranged side by side in a longitudinal direction of the first band body.

## Description

### Technical Field

The present invention relates to a hemostatic device.

### Background Art

As one of catheter procedures, there is known a procedure in which various medical elongated bodies are introduced into a blood vessel of a limb such as an arm or hand of a patient through a puncture site formed by puncturing the blood vessel to perform a diagnosis or a therapy at a lesion site. For example, Patent Literature 1 discloses a hemostatic device for stopping bleeding at a puncture site formed to enable access to a blood vessel (including a distal radial artery) running in a hand.

The hemostatic device disclosed in Patent Literature 1 includes a pressing member that applies a compressive force to a puncture site (hereinafter, also simply referred to as a "puncture site") formed in a hand of a patient, a cover member on which the pressing member is disposed, and a plurality of band bodies extending from the cover member for securing the pressing member to the hand of the patient. The plurality of band bodies includes a band body (referred to as a "first band body") disposed so as to be hooked on an interdigital space between fingers and a band body (referred to as a "second band body" and a "third band body") disposed along a circumferential direction of the hand.

### Citation List

### Patent Literature

Patent Literature 1: JP 2022-175655 A

### Summary of Invention

### Technical Problem

When stopping bleeding at the puncture site formed in the limb of the patient, an operator such as a doctor (hereinafter, referred to as "operator") secures the hemostatic device to the hand of the patient such that the cover member is placed to cover the puncture site using the band bodies. The hemostatic device can stop bleeding at the puncture site as the pressing member applies a compressive force to the puncture site in a state where the pressing member is placed at the puncture site.

When securing the hemostatic device to the hand using the band bodies, the operator wraps, for example, the second band body and the third band body along the circumferential direction of the hand to secure the hemostatic device. In a state where the second band body and the third band body are secured to the hand, the operator passes the first band body through the interdigital space and secures the first band body to the other band bodies. By securing the hemostatic device to the hand of the patient using three band bodies as described above, the operator can prevent the hemostatic device and the pressing member from being displaced while applying a compressive force to the puncture site by the hemostatic device.

When stopping bleeding at the puncture site of the hand using the hemostatic device having a plurality of band bodies in the manner described above, the operator needs to be sufficiently careful not to make an error in the arrangement of each band body, the method for securing each band body, and the like. For example, in the hemostatic device described in Patent Literature 1, each band body is provided with a symbol (number) indicating an order of securing each band body to the hand. When placing the hemostatic device on the hand of the patient, the operator places and secures the band bodies to the hand of the patient according to the order indicated by the symbols attached to the band bodies while visually confirming the symbols, thereby being capable of easily and smoothly carrying out the work to place the hemostatic device on the hand of the patient.

For example, hemostatic devices used for stopping bleeding at a puncture site of a hand include a device configured to be placeable on both the right hand and the left hand of a patient. When the hemostatic device having such a structure is used for stopping bleeding at puncture sites of the right hand and the left hand, the pressing member and the band bodies are commonly used between the right hand and the left hand. However, in a case where the hemostatic device is selectively used for the right hand or the left hand, positions where the pressing member and the band bodies are placed are different between the right hand and the left hand. Therefore, the operator needs to ascertain whether or not the hemostatic device is appropriately placed in each case of stopping bleeding at the puncture site in the right hand and stopping bleeding at the puncture site in the left hand. However, the conventional hemostatic device is not provided with an index or the like for confirming an appropriate placement state of the hemostatic device according to each hand when being used for either the right hand or the left hand. Therefore, when placing the hemostatic device on the right hand or the left hand of the patient, the operator cannot confirm whether or not the hemostatic device is appropriately placed for each hand, and thus, may spend a large amount of labor to place the hemostatic device.

The present invention has been made in view of the above problems, and an object of the present invention is to provide a hemostatic device capable of easily ascertaining whether or not a pressing member and each band body are appropriately placed when used for hemostasis of a puncture site formed in the right hand and the left hand of a patient.

### Solution to Problem

The present invention is achieved by any one of the following aspects (1) to (8).

(1) A hemostatic device that is placeable for both a right hand and a left hand of a patient according to a position of a puncture site of the patient, the hemostatic device comprising: a cover member configured to cover the puncture site; and a pressing member connected to the cover member and configured to compress the puncture site, wherein the cover member includes a main body where the pressing member is located, a first band body configured to be placed between fingers of the patient and extending in a first direction from the main body, a second band body extending in a second direction different from the first direction from the main body, a third band body facing the second band body across the pressing member and extending from the main body in a third direction different from the first direction and the second direction, and a layout display portion showing a placement example of the hemostatic device for the right hand and the left hand of the patient, the pressing member and the layout display portion are positioned on a center line of the first band body, the layout display portion includes a first graphic portion showing a placement example of the hemostatic device for the right hand of the patient and a second graphic portion showing a placement example of the hemostatic device for the left hand of the patient, and the first graphic portion and the second graphic portion are arranged side by side in a longitudinal direction of the first band body.
(2) The hemostatic device according to (1), wherein the first band body includes a first end connected to the main body, a second end located opposite to the first end, and an intermediate portion extending between the first end and the second end, and the layout display portion is located on a side of the first end of the intermediate portion of the first band body.
(3) The hemostatic device according to (1) or (2), wherein the first band body is configured to be more opaque than the main body, and at least a part of the layout display portion has a color different from a color of the first band body.
(4) The hemostatic device according to (3), wherein the first band body includes an inner surface on a side where the pressing member is located and an outer surface located on a side opposite to the inner surface, and the layout display portion is disposed only on the outer surface.
(5) The hemostatic device according to any one of (1) to (4), wherein the pressing member has an elliptical shape having a long axis in the longitudinal direction of the first band body, the first graphic portion includes positions of the pressing member and the layout display portion in a state where the hemostatic device is worn on the right hand, and the second graphic portion includes positions of the pressing member and the layout display portion in a state where the hemostatic device is worn on the left hand.
(6) The hemostatic device according to (2), wherein the pressing member includes an inflatable member inflatable by injection of a fluid, the inflatable member is connected to an injection member for injecting the fluid into the inflatable member by means of a tube member, the first band body has a slit extending in the longitudinal direction of the first band body, the tube member is inserted from an inner surface side of the first band body toward an outer surface side of the first band body through the slit, and the layout display portion is located on a side of the second end of the first band body with respect to an end of the slit located on a side of the first end of the first band body.
(7) The hemostatic device according to any one of (1) to (6) comprising a support member disposed on the cover member so as to cover the pressing member, wherein the main body has a two-layer structure including a holding portion that holds the support member, the holding portion has an upper surface region located on an upper surface side of the support member, a lower surface region located on a lower surface side of the support member and facing the upper surface region across the support member, and a curved region connecting the upper surface region and the lower surface region on a side of a proximal portion of the support member, and the first band body is connected to the main body at a position facing the curved region across the support member.
(8) The hemostatic device according to (7), wherein the curved region is formed by folding back a part of the main body toward the first band body, the lower surface region has a constricted portion formed on a side of the curved region, and the constricted portion is configured in such a manner that a width of the lower surface region decreases from the first band body toward the curved region.

### Advantageous Effects of Invention

The hemostatic device described above is configured to be placeable on both the right hand and the left hand of the patient, whereby an operator such as a doctor can freely select the placement on the right hand and the placement on the left hand of the patient according to the puncture site formed in the hand of the patient. Therefore, the operator can treat both the right hand and the left hand of the patient with one hemostatic device, whereby convenience at the time of use is improved. In addition, the first band body included in the cover member of the hemostatic device is provided with a layout display portion showing a placement example of the hemostatic device for the right hand and the left hand of the patient. The layout display portion includes a first graphic portion showing a placement example of the hemostatic device for the right hand of the patient and a second graphic portion showing a placement example of the hemostatic device for the left hand of the patient. When placing the hemostatic device on the hand of the patient, the operator visually checks the first graphic portion and the second graphic portion provided on the first band body, thereby being capable of easily ascertaining whether or not the pressing member and the band bodies included in the hemostatic device are appropriately placed on the hand. In addition, the first graphic portion and the second graphic portion are positioned on the center line of the first band body and are arranged side by side in the longitudinal direction of the first band body, whereby the graphic portions can be provided to be noticeable in a relatively wide range in the width direction orthogonal to the longitudinal direction of the first band body. In addition, since the first graphic portion and the second graphic portion are positioned on the center line of the first band body and are arranged side by side in the longitudinal direction of the first band body as described above, it is possible to prevent the first graphic portion and the second graphic portion from being entirely hidden outside the visual field of the operator even if the first band body is twisted when the operator places the hemostatic device on the hand of the patient. Therefore, even in a case where the first band body is twisted as described above, the operator can easily and appropriately place the hemostatic device on the hand of the patient in accordance with the content indicated by the layout display portion.

### Brief Description of Drawings

Fig. 1 is a plan view illustrating a hemostatic device according to an embodiment as viewed from an outer surface side of each band body.
Fig. 2 is a plan view illustrating the hemostatic device according to the embodiment as viewed from an inner surface side of each band body.
Fig. 3 is an enlarged view illustrating a part of the hemostatic device as viewed from an outer surface side of a main body of a cover member.
Fig. 4 is an enlarged view illustrating a part of the hemostatic device as viewed from an inner surface side of the main body of the cover member.
Fig. 5 is a partial cross-sectional view of the hemostatic device taken along a line 5A-5A illustrated in Fig. 3, and illustrates a state when an inflatable member is inflated.
Fig. 6 is a partial cross-sectional view of the hemostatic device taken along a line 6A-6A illustrated in Fig. 3, and illustrates a state when the inflatable member is inflated.
Fig. 7 is an enlarged view of a layout display portion as viewed from an outer surface side of a first band body.
Fig. 8 is an enlarged view of the layout display portion as viewed from an inner surface side of the first band body.
Fig. 9 is a diagram illustrating a hand (right hand) of a patient for which the hemostatic device according to the embodiment is to be used.
Fig. 10 is a diagram schematically illustrating a first usage example of the hemostatic device according to the embodiment.
Fig. 11 is a diagram schematically illustrating the first usage example of the hemostatic device according to the embodiment.
Fig. 12 is an enlarged view of a section enclosed by a broken line 12A illustrated in Fig. 11.
Fig. 13 is a diagram schematically illustrating the first usage example of the hemostatic device according to the embodiment.
Fig. 14 is a partial cross-sectional view taken along a line 14A-14A illustrated in Fig. 13.
Fig. 15 is a partial cross-sectional view taken along a line 15A-15A illustrated in Fig. 13.
Fig. 16 is a diagram as viewed in a direction of an arrow 16A illustrated in Fig. 13.
Fig. 17 is a diagram schematically illustrating a second usage example of the hemostatic device according to the embodiment.
Fig. 18 is an enlarged view of a section enclosed by a broken line 18A illustrated in Fig. 17.
Fig. 19 is a diagram schematically illustrating the second usage example of the hemostatic device according to the embodiment.

### Description of Embodiments

An embodiment of the present invention will be described below with reference to the accompanying drawings. The following description does not limit the technical scope or the significance of each term set forth in the claims. The dimensional ratios in the drawings are exaggerated for convenience of description and may be different from actual ratios.

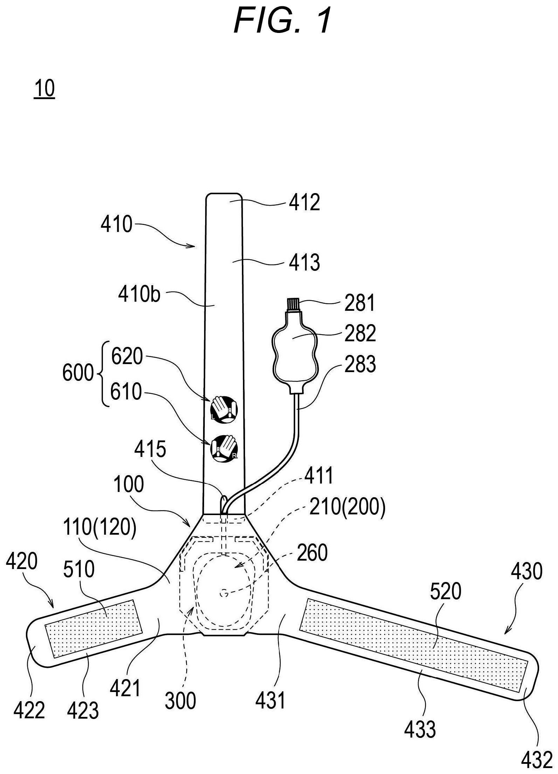

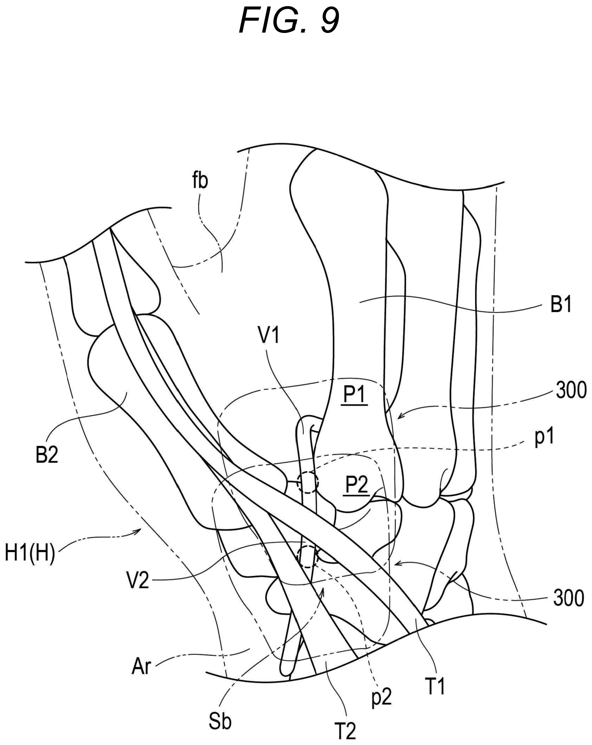

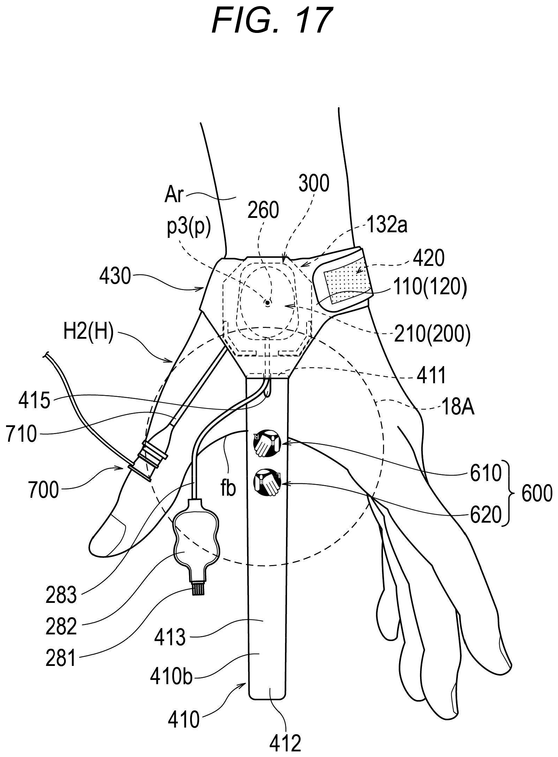

Figs. 1 to 8 are diagrams for describing a hemostatic device 10 according to an embodiment. Figs. 9 to 16 are diagrams for describing a first usage example in which the hemostatic device 10 is used for a right hand H1. Figs. 17 to 19 are diagrams for describing a second usage example in which the hemostatic device 10 is used for a left hand H2.

As illustrated in Figs. 9, 13, 14, 15, 19, and the like, for example, the hemostatic device 10 can be used to stop bleeding at a puncture site p1 formed in a hand H located on a distal side (fingertip side) with respect to a forearm Ar of a patient when a sheath tube 710 of an introducer 700 placed at the puncture site p is removed.

The specific position of the puncture site where bleeding is to be stopped by the hemostatic device 10 is not particularly limited, but in the present specification, the first puncture site p1, a second puncture site p2, and a third puncture site p3 are described as an example of the puncture site.

As illustrated in Figs. 9, 10, 11, 13, 14, and 15, the first puncture site p1 is a puncture site formed in a distal radial artery (hereinafter, also referred to as "blood vessel V1") located on a distal side with respect to a snuff box Sb of a palmar artery running on the dorsal side of a right hand H1 of the patient (hereinafter, also simply referred to as "right hand H1") located on the distal side with respect to the forearm Ar of the patient. In addition, the first puncture site p1 is located at a predetermined position, which is a position between a metacarpal bone B1 of an index finger and a metacarpal bone B2 of a thumb and avoiding the metacarpal bone B1 of the index finger and the metacarpal bone B2 of the thumb. Note that the snuff box Sb is a cavity in the hand located in the vicinity of the radius when the patient spreads the thumb of the hand H.

As illustrated in Fig. 9, the second puncture site p2 is a puncture site formed in an artery V2 located in the snuff box Sb of the palmar artery running on the dorsal side of the right hand H1. The second puncture site p2 is located on the forearm Ar side (proximal side) of the patient with respect to the first puncture site p1, and is located between an extensor pollicis longus muscle tendon T1 and an extensor pollicis brevis muscle tendon T2 located at the dorsal side of the right hand H1 of the patient. In addition, the second puncture site p2 is located at a predetermined position avoiding the position of the metacarpal bone B1 of the index finger and the position of the metacarpal bone B2 of the thumb.

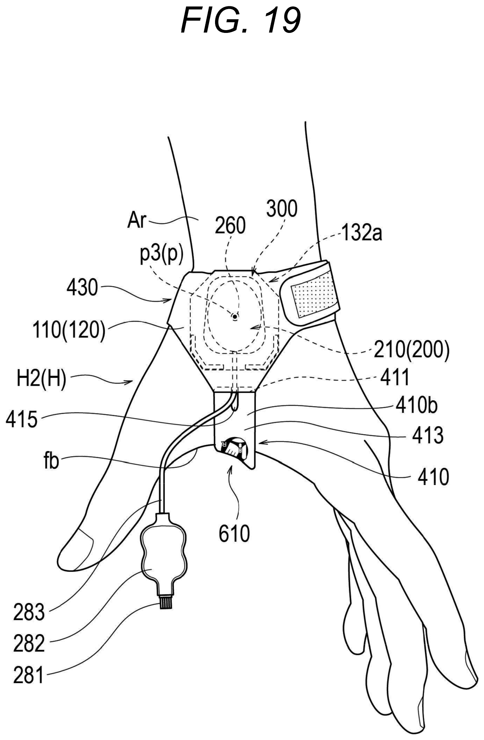

The hemostatic device 10 can also be applied to hemostasis of the third puncture site p3 on the left hand H2 of the patient (hereinafter, simply referred to as "left hand H2") formed at a position corresponding to the first puncture site p1 on the right hand H1 of the patient as illustrated in Figs. 17 and 19. Note that the hemostatic device 10 can also be applied to hemostasis of a puncture site on the left hand H2 of the patient formed at a position corresponding to the second puncture site p2 on the right hand H1 of the patient, although this is not described in the present specification.

Hereinafter, the hemostatic device 10 will be described in detail.

### <Hemostatic Device 10>

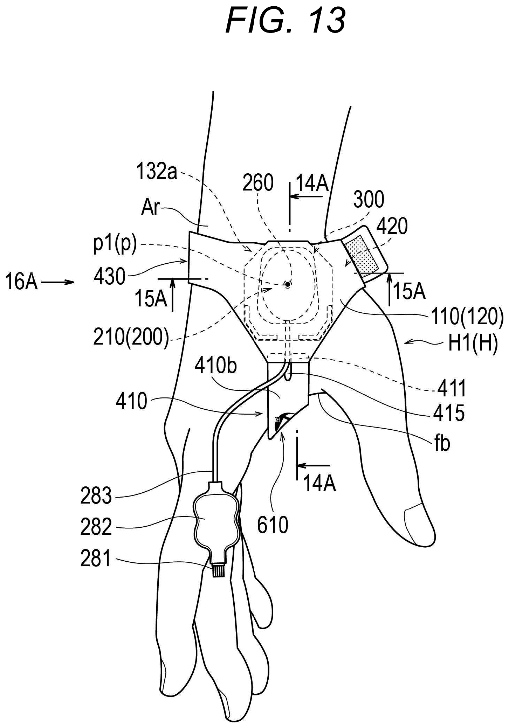

As illustrated in Figs. 13 and 19, the hemostatic device 10 is configured to be placeable on both the right hand H1 and the left hand H2 of the patient according to the position of the puncture site p of the patient.

In the following description, the present embodiment will be described by way of an example of a case where the hemostatic device 10 is used for hemostasis of the first puncture site p1 of the right hand H1.

As illustrated in Figs. 1, 2, 9, 13, 14, and 15, the hemostatic device 10 includes a cover member 100 configured to cover the first puncture site p1 and a pressing member 200 connected to the cover member 100 and configured to compress the first puncture site p1.

In the present specification, the fingertip side of a finger in the hemostatic device 10 is defined as a "distal side", and the forearm Ar side of the hemostatic device 10 is defined as a "proximal side" in a state where the hemostatic device 10 is worn on the right hand H1 (or the left hand H2) of the patient.

### <Pressing Member 200>

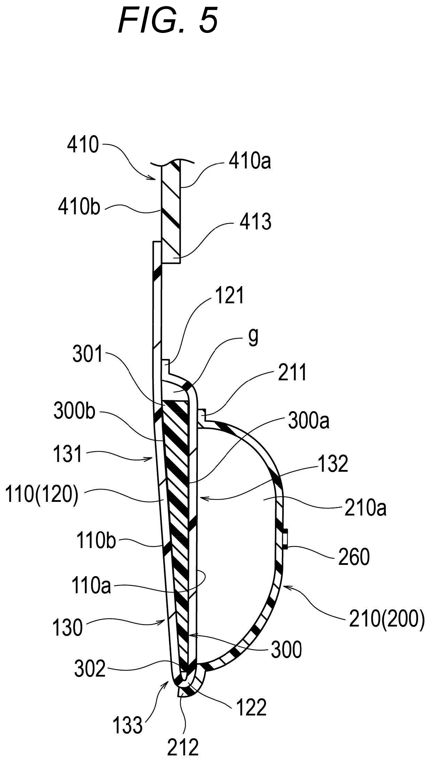

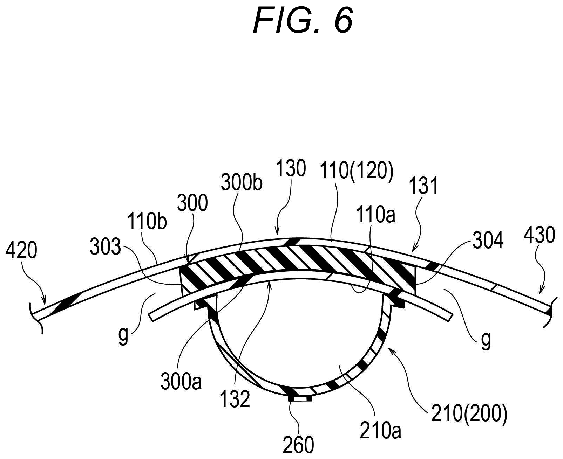

The pressing member 200 can be constituted by, for example, an inflatable member 210 that can be inflated by injection of a fluid as illustrated in Figs. 5 and 6. In the present embodiment, an example in which the pressing member 200 is constituted by the inflatable member 210 will be described in the following description.

The inflatable member 210 includes an inner cavity 210a into which the fluid can be injected. The inflatable member 210 is configured to be inflated as the fluid is injected into the inner cavity 210a and deflated as the fluid injected into the inner cavity 210a is discharged. The fluid used for the inflation of the inflatable member 210 is, for example, a gas such as air. Figs. 5 and 6 illustrate cross-sectional views of the inflatable member 210 in an inflated state.

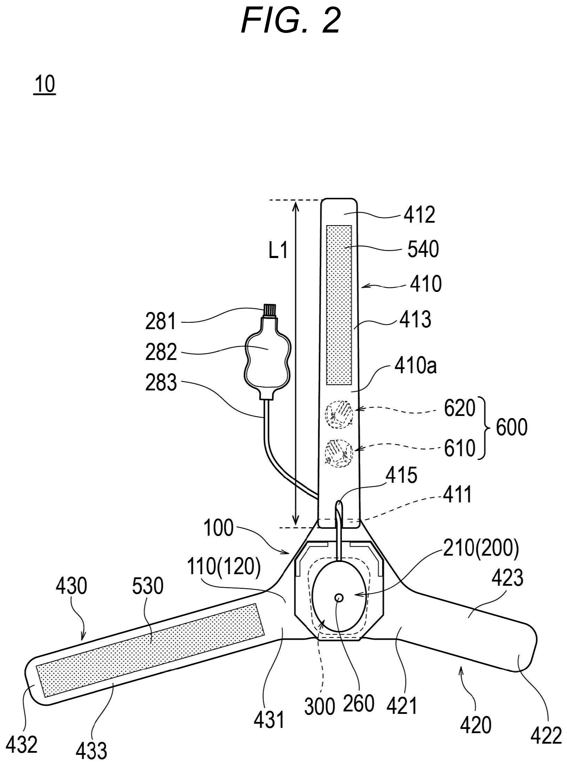

The tube member 283 (see Figs. 1 and 2) to be described later is connected to the inner cavity 210a of the inflatable member 210.

As illustrated in Figs. 5 and 6, the inflatable member 210 can include, for example, a film-shaped member (sheet material) connected to a sheet material 120 that constitutes a main body 110 of the cover member 100.

The inflatable member 210 can be disposed to form an inner cavity 210a with a lower surface region 132 of a holding portion 130 formed by the sheet material 120.

An outer peripheral edge of the film-shaped member forming the inflatable member 210 defines an edge of the inflatable member 210. The edge of the inflatable member 210 can be connected to the sheet material 120 by, for example, fusing, using an adhesive, or the like.

As illustrated in Fig. 5, a section 211 of an edge of the inflatable member 210 located at an upper end portion 301 side of the support member 300 can be fixed to the sheet material 120 at a position overlapping a lower surface 300a of the support member 300 in the plane direction. In addition, a section 212 of the edge of the inflatable member 210 located at a lower end portion 302 side of the support member 300 can be connected to a curved region 133 formed near a proximal portion 122 of the cover member 100.

The film-shaped member constituting the inflatable member 210 can be made of, for example, a resin material having a predetermined thickness.

A material for the film-shaped member constituting the inflatable member 210 is not particularly limited, and examples thereof include polyvinyl chloride, ethylene-vinyl acetate copolymer (EVA), polyvinylidene chloride, silicone, polyamide (nylon), polyurethane, polyolefin such as polyethylene, polypropylene, or polybutadiene, polyester such as polyethylene terephthalate (PET) or polybutylene terephthalate (PBT), various thermoplastic elastomers such as polyamide elastomer (nylon elastomer), polyurethane elastomer, or polyester elastomer, or any combination thereof (e.g., blended resin, polymer alloy, laminate, or the like).

As illustrated in Figs. 5 and 6, the inflatable member 210 is disposed on an inner surface 110a side of the main body 110 of the cover member 100.

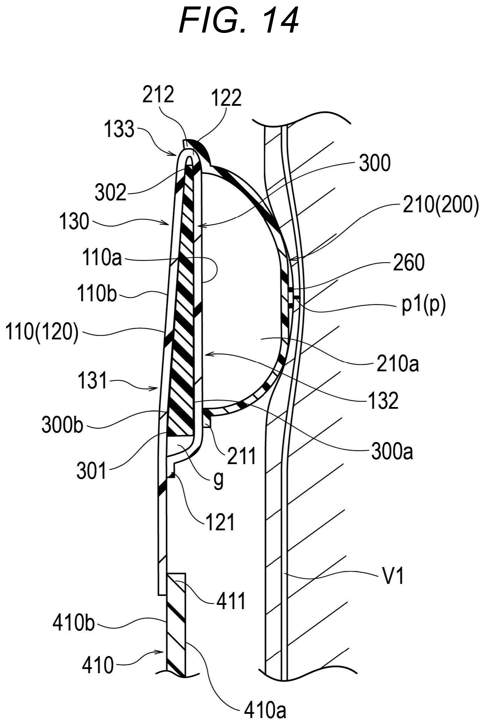

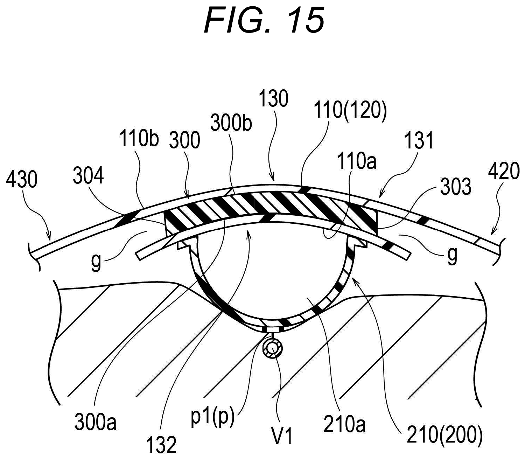

In the present embodiment, the inner surface 110a of the main body 110 is defined by a surface to be placed on a body surface side of the right hand H1 in the lower surface region 132 of the holding portion 130 (see Figs. 14 and 15). An outer surface 110b of the main body 110 is defined by a surface on a side facing the outside in an upper surface region 131 of the holding portion 130.

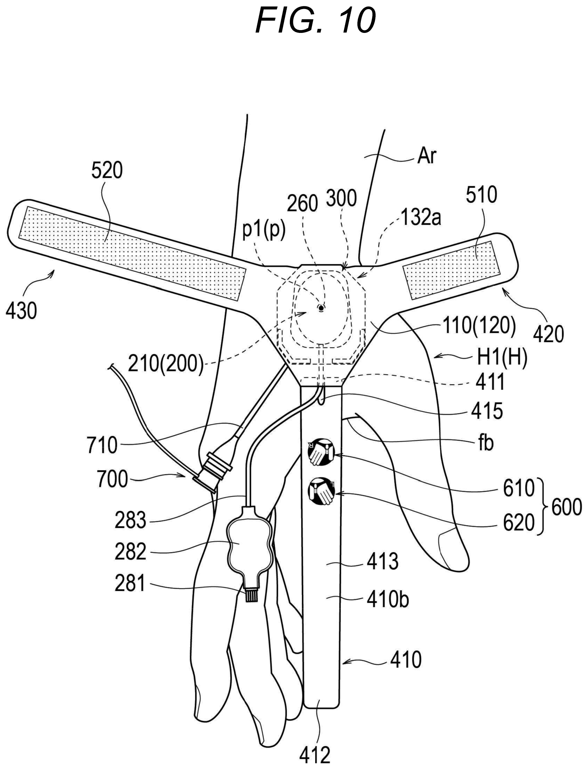

The inflatable member 210 includes a marker portion 260 for aligning the inflatable member 210 with the first puncture site p1 as illustrated in Figs. 10, 11, and 13.

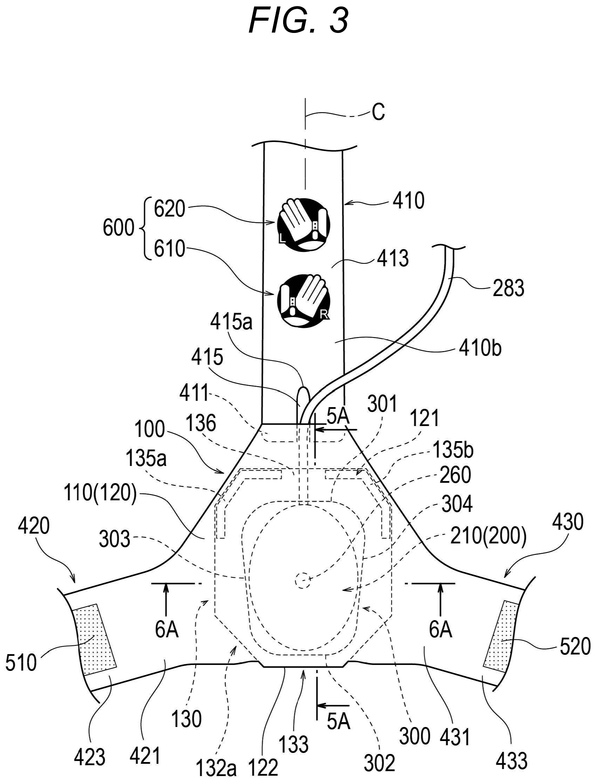

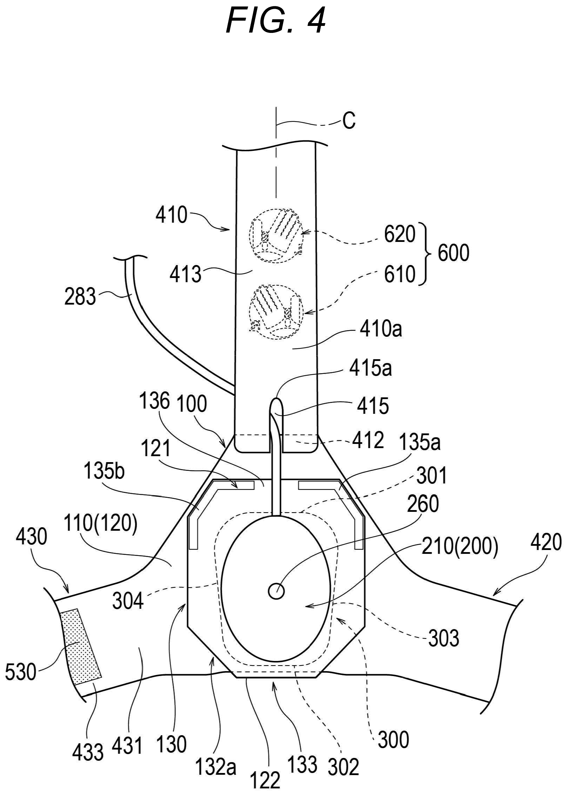

As illustrated in Figs. 3 and 4, the marker portion 260 can include a transparent central part and a colored circular frame part surrounding the central part.

The marker portion 260 is provided on an outer surface of the inflatable member 210 as illustrated in Figs. 5 and 6. The marker portion 260 may be provided on, for example, an inner surface of the inflatable member 210 facing the inner cavity 210a.

The specific shape and color, a formation method, and the like of the marker portion 260 are not particularly limited. The marker portion 260 can also be constituted by, for example, a circular marker that is entirely colored, a marker including a transparent central part and a rectangular frame part, a rectangular marker that is entirely colored, or the like.

The inflatable member 210 can be configured to have an elliptical shape having a major axis in the longitudinal direction of the first band body 410 in plan view illustrated in Figs. 3 and 4. However, the planar shape of the inflatable member 210 in plan view is not particularly limited. The inflatable member 210 may be configured to have a planar shape such as a circular shape, a rectangular shape, or an oval shape having a major axis in the longitudinal direction of the first band body 410.

The specific configuration of the inflatable member 210 included in the hemostatic device 10 is not particularly limited. The inflatable member 210 may be formed in such a manner that, for example, in a state where the inner cavity 210a is formed between two sheet-shaped members, the edge of the other of the sheet-shaped members is joined to the edge of one of the sheet-shaped members. In a case where the inflatable member 210 is formed as described above, the surface of the one of the sheet-shaped members of the inflatable member 210 can be connected to the sheet material 120 constituting the main body 110 of the cover member 100. In addition, the inflatable member 210 can also be constituted by, for example, one bag-shaped member shaped to include the inner cavity 210a inside. In a case where the inflatable member 210 is formed as described above, a part of the surface of the bag-shaped member of the inflatable member 210 can be connected to the sheet material 120 constituting the main body 110 of the cover member 100.

### <Cover Member 100>

As illustrated in Figs. 3, 4, 10, 11, and 13, the cover member 100 includes the main body 110 in which the inflatable member 210 is located, a first band body 410 that is configured to be placed between fingers of the patient and extends from the main body 110 in a first direction, a second band body 420 extending from the main body 110 in a second direction different from the first direction, a third band body 430 facing the second band body 420 across the inflatable member 210 and extending from the main body 110 in a third direction different from the first direction and the second direction, and a layout display portion 600 that shows placement examples of the hemostatic device 10 for the right hand H1 and the left hand H2.

A region of the cover member 100 where the inflatable member 210 is disposed (a region overlapping the inflatable member 210 in the plan views illustrated in Figs. 3 and 4) defines the main body 110.

As illustrated in Figs. 5 and 6, the main body 110 has a two-layer structure. The main body 110 having a two-layer structure includes the holding portion 130 that holds the support member 300.

As illustrated in Fig. 5, the holding portion 130 includes the upper surface region 131 located on the upper surface 300b side of the support member 300, the lower surface region 132 that is located on the lower surface 300a side of the support member 300 and faces the upper surface region 131 across the support member 300, and the curved region 133 connecting the upper surface region 131 and the lower surface region 132 on the proximal side of the support member 300 (the lower end portion 302 side of the support member 300).

The curved region 133 is formed by folding back a part of the sheet material 120 constituting the main body 110 toward the first band body 410. In the present embodiment, a part of the sheet material 120 constituting the main body 110 is folded back from the proximal side to the distal side of the main body 110. A clearance g in which the support member 300 can be disposed is formed between the upper surface region 131 and the lower surface region 132 of the sheet material 120 folded back as described above. A section of the sheet material 120 where the clearance g is formed constitutes the holding portion 130 for holding the support member 300.

As illustrated in Figs. 3 and 4, in the sheet material 120, the first end 121 disposed on the upper end portion 301 side of the support member 300 and a predetermined range from the first end 121 toward the proximal portion 122 are connected to the sheet material 120 at a position on the first band body 410 side with respect to the upper end portion 301 of the support member 300. The first end 121 and the predetermined range from the first end 121 toward the proximal portion 122 can be fixed to the sheet material 120 with fused portions 135a and 135b, for example.

As illustrated in Figs. 3 and 4, the vicinity of the central part of the first end 121 in the width direction in plan view can be defined as, for example, a non-connected portion 136 that is not connected to the sheet material 120. In addition, the ends of the fused portions 135a and 135b on the proximal portion 122 side can be disposed at predetermined positions on the first end 121 side with respect to the proximal portion 122 of the sheet material 120.

As illustrated in Fig. 5, the proximal portion 122 of the cover member 100, which is formed of a part of the sheet material 120, is located in the curved region 133.

As illustrated in Fig. 5, the clearance g of the holding portion 130 is closed in the longitudinal width direction (vertical direction in Figs. 3 and 4) of the inflatable member 210. In addition, as illustrated in Fig. 6, a part of the clearance g of the holding portion 130 communicates with the outside on the second band body 420 side and the third band body 430 side in the lateral width direction (lateral direction in Figs. 3 and 4) of the inflatable member 210.

As illustrated in Figs. 3 and 4, the lower surface region 132 has a constricted portion 132a located on the curved region 133 side.

The constricted portion 132a is configured such that a width (lateral width) of the lower surface region 132 decreases from the first band body 410 side to the curved region 133 side. That is, the constricted portion 132a is constituted by a section where the width of the lower surface region 132 gradually decreases from the distal side to the proximal side in the plan views illustrated in Figs. 3 and 4.

The constricted portion 132a can be configured such that, for example, the width decreases toward the curved region 133 at substantially the same rate in a bilaterally symmetric manner with respect to a center line C of the first band body 410 as illustrated in Figs. 3 and 4.

### <First Band Body 410, Second Band Body 420, Third Band Body 430>

The first band body 410 is connected to the main body 110 at a position facing the curved region 133 across the support member 300 as illustrated in Figs. 3 and 4. The first band body 410 can be connected to the main body 110 by, for example, fusion or adhesion.

The second band body 420 and the third band body 430 are integrally formed with the main body 110.

Note that, similar to the first band body 410, the second band body 420 and the third band body 430 may be formed of a member different from the main body 110 and connected to the main body 110. In addition, the first band body 410 may be formed integrally with the main body 110 similarly to the second band body 420 and the third band body 430.

As illustrated in Figs. 1, 2, 3, and 4, the first band body 410 includes a first end 411 connected to the main body 110 of the cover member 100, a second end 412 located opposite to the first end 411, and an intermediate portion 413 extending between the first end 411 and the second end 412.

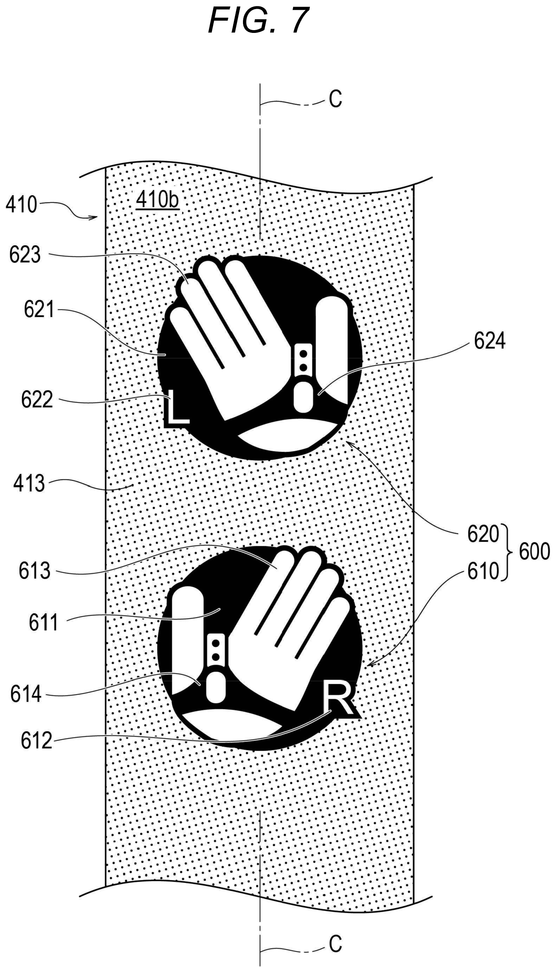

In the present specification, a direction (vertical direction in Fig. 7) parallel to the direction in which the intermediate portion 413 of the first band body 410 extends is defined as a "longitudinal direction of the first band body 410". In addition, a direction (lateral direction in Fig. 7) orthogonal to the longitudinal direction of the first band body 410 is defined as a "width direction of the first band body 410". The center line C of the first band body 410 is defined by a straight line passing through a substantially central position in the width direction of the first band body 410 and extending in the longitudinal direction of the first band body 410.

As illustrated in Figs. 3 and 4, the first band body 410 has a slit 415 extending in the longitudinal direction of the first band body 410.

The slit 415 penetrates an inner surface 410a and an outer surface 410b of the first band body 410.

As illustrated in Figs. 3 and 4, the tube member 283 of the injection member 281 is inserted from the inner surface 410a side of the first band body 410 toward the outer surface 410b side of the first band body 410 through the slit 415. Therefore, as illustrated in Figs. 1 and 2, the injection member 281 and a buffer member 282 which are connected to the tube member 283 are located on the outer surface 410b side of the first band body 410 together with a part of the tube member 283.

As illustrated in Fig. 3, the slit 415 is positioned on the center line C of the first band body 410 together with the layout display portion 600. Therefore, when placing the hemostatic device 10 on the right hand H1 of the patient, the operator can easily prevent the tube member 283 from covering the layout display portion 600 by moving the tube member 283 included in the injection member 281 to the outside of the first band body 410. Note that the wording "positioned on the center line C" means that at least a part of the slit 415 is positioned on the center line C (on the extension of the center line C). Therefore, the center of the slit 415 in the width direction may be shifted from the center line C to one side or the other side in the width direction of the first band body 410. However, the position of the slit 415 is not particularly limited.

The slit 415 can be configured to extend, for example, by a predetermined length from the first end 411 of the first band body 410 along the longitudinal direction of the first band body 410.

As illustrated in Fig. 13, the first band body 410 can be placed so as to be hooked on an interdigital space fb located between the thumb and the index finger of the right hand H1 of the patient in a state where the inflatable member 210 is placed at the first puncture site p1.

As illustrated in Figs. 1, 2, 3, and 4, the second band body 420 includes a first end 421 connected to the main body 110 of the cover member 100, a second end 422 located opposite to the first end 421, and an intermediate portion 423 extending between the first end 421 and the second end 422.

The second direction in which the second band body 420 extends is not particularly limited as long as it is a direction different from the first direction in which the first band body 410 extends.

As illustrated in Figs. 1, 2, 3, and 4, the third band body 430 includes a first end 431 integrally connected to the main body 110 of the cover member 100, a second end 432 located opposite to the first end 431, and an intermediate portion 433 extending between the first end 431 and the second end 432.

The third direction in which the third band body 430 extends is not particularly limited as long as it is different from the first direction in which the first band body 410 extends and the second direction in which the second band body 420 extends.

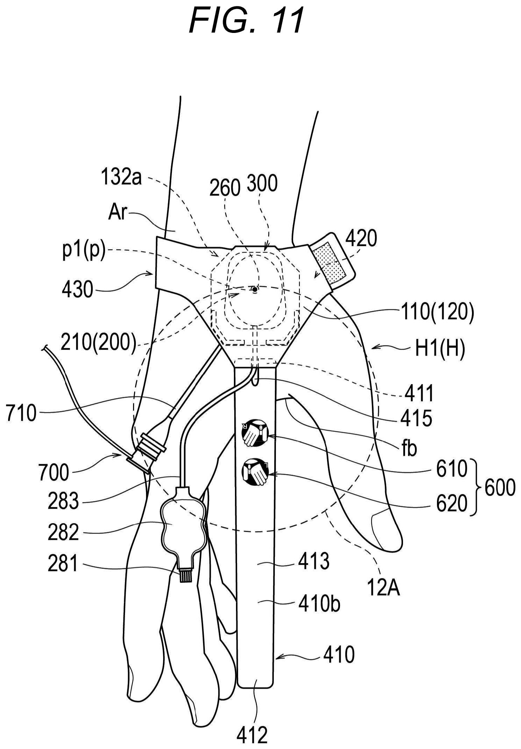

As illustrated in Figs. 10 and 11, the second band body 420 and the third band body 430 can be placed so as to be wrapped along the outer periphery of the right hand H1 when the hemostatic device 10 is worn on the right hand H1 of the patient. As illustrated in Figs. 3 and 4, the second band body 420 and the third band body 430 are located at positions facing each other across the inflatable member 210. Therefore, when the second band body 420 and the third band body 430 are wrapped around the right hand H1, a tensile force in a direction of separating the second band body 420 and the third band body 430 from each other is applied to the both, and a tensile force in the same direction is also applied to the main body 110.

The constituent material of each of the band bodies 410, 420, and 430 is not particularly limited, and each of the band bodies 410, 420, and 430 can be made of, for example, a vinyl chloride resin, a polyurethane resin, a polyester resin, or the like. Furthermore, a shape, a length, a thickness, and the like of each of the band bodies 410, 420, and 430 are not particularly limited.

Four securing parts, that is, a first securing part 510, a second securing part 520, a third securing part 530, and a fourth securing part 540, which enable the cover member 100 to be secured on the right hand H1, are disposed in the band bodies 410, 420, and 430, respectively.

As illustrated in Fig. 1, the first securing part 510 is provided on an outer surface of the intermediate portion 423 of the second band body 420.

As illustrated in Fig. 1, the second securing part 520 is provided on an outer surface of the intermediate portion 433 of the third band body 430.

As illustrated in Fig. 2, the third securing part 530 is provided on an inner surface of the intermediate portion 433 of the third band body 430.

As illustrated in Fig. 2, the fourth securing part 540 is provided on an inner surface of the intermediate portion 413 of the first band body 410.

The first securing part 510 and the second securing part 520 can be constituted by a male side of a hook-and-loop fastener. The third securing part 530 and the fourth securing part 540 can be constituted by a female side of a hook-and-loop fastener. The hook-and-loop fastener in the present specification is a fastener that allows surfaces to be attached and detached, and is, for example, Magic Tape (registered trademark) or Velcro (registered trademark).

The second band body 420 and the third band body 430 are configured to be attachable and detachable by means of the first securing part 510 located on the outer surface of the intermediate portion 423 of the second band body 420 and the third securing part 530 located on the inner surface of the intermediate portion 433 of the third band body 430. The first band body 410 and the third band body 430 are configured to be attachable and detachable via the fourth securing part 540 located on the inner surface of the intermediate portion 413 of the first band body 410 and the second securing part 520 located on the outer surface of the intermediate portion 433 of the third band body 430.

Note that a specific structure of each of the securing parts 510, 520, 530, and 540 is not limited as long as the cover member 100 can be secured on the hand H by connecting the band bodies 410, 420, and 430 to each other in a state where the hemostatic device 10 is worn on the hand H (right hand H1 or left hand H2). For example, it is possible to freely eliminate some of the securing parts or to freely change the position where the securing part is provided in each of the band bodies 410, 420, and 430. Furthermore, in a case where each of the securing parts 510, 520, 530, and 540 is formed of the hook-and-loop fastener, the male side and the female side of the hook-and-loop fastener may be interchanged. In addition, the securing parts 510, 520, 530, and 540 may be configured using, for example, a coupling mechanism or the like including a frame part provided with a snap, a button, a clip, or a protrusion and an engaged part provided with a hole engageable with the frame part.

### <Layout Display Portion 600>

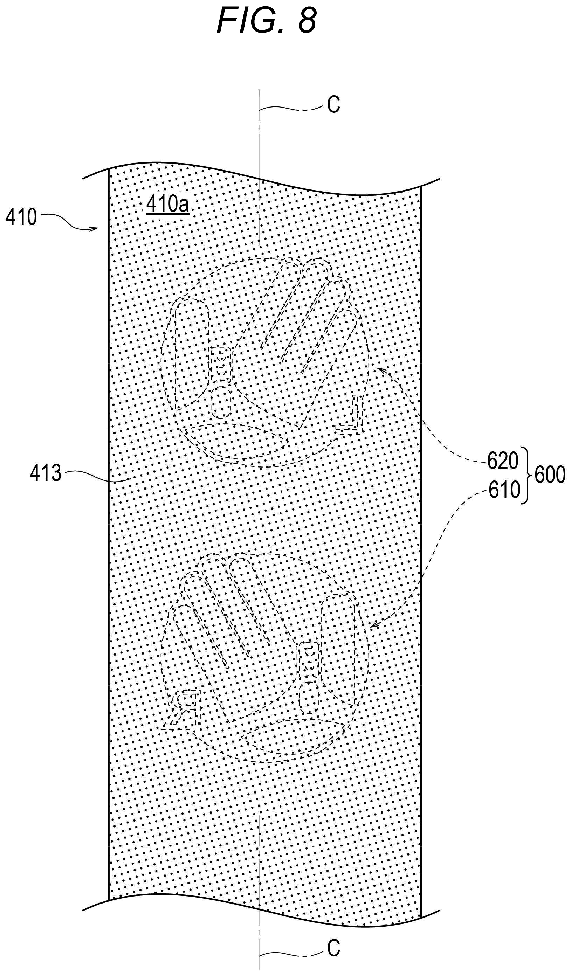

Figs. 7 and 8 illustrate the enlarged layout display portion 600 disposed on the first band body 410. Fig. 7 illustrates the layout display portion 600 as viewed from the outer surface 410b side of the first band body 410. Fig. 8 illustrates the layout display portion 600 as viewed from the inner surface 410a side of the first band body 410.

As illustrated in Figs. 3, 4, 7, and 8, the inflatable member 210 and the layout display portion 600 are located on the center line C of the first band body 410.

In the present embodiment, the center position of the inflatable member 210 in the width direction, the center position of a first graphic portion 610 in the width direction, and the center position of a second graphic portion 620 in the width direction are all disposed on the center line C.

Note that the wording "positioned on the center line C" means that at least a part of the inflatable member 210 is positioned on the center line C (on the extension of the center line C) and that at least a part of the layout display portion 600 (first graphic portion 610 and second graphic portion 620) is positioned on the center line C. Therefore, the center position in the width direction of the inflatable member 210, the center position in the width direction of the first graphic portion 610, and the center position in the width direction of the second graphic portion 620 may be shifted from the center line C to one side or the other side in the width direction of the first band body 410.

As illustrated in Figs. 7 and 8, the layout display portion 600 includes the first graphic portion 610 showing a placement example of the hemostatic device 10 for the right hand H1 of the patient and the second graphic portion 620 showing a placement example of the hemostatic device 10 for the left hand H2 of the patient.

As illustrated in Figs. 7 and 8, the first graphic portion 610 and the second graphic portion 620 are arranged side by side in the longitudinal direction of the first band body 410. The first graphic portion 610 is disposed on the first end 411 side of the first band body 410 with respect to the second graphic portion 620.

The first graphic portion 610 and the second graphic portion 620 can be spaced from each other at a predetermined distance in the longitudinal direction of the first band body 410. Since there is a distance between the first graphic portion 610 and the second graphic portion 620, the operator can easily recognize the boundary between the first graphic portion 610 and the second graphic portion 620 when viewing the layout display portion 600. As a result, the operator can clearly distinguish the first graphic portion 610 and the second graphic portion 620 when viewing the layout display portion 600.

Note that a specific distance between the first graphic portion 610 and the second graphic portion 620 is not particularly limited. For example, the distance between the first graphic portion 610 and the second graphic portion 620 can be set to about 3 mm along the longitudinal direction of the first band body 410.

As illustrated in Figs. 1, 2, 3, and 4, the layout display portion 600 is located on the first end 411 side of the intermediate portion 413 of the first band body 410.

The above wording "on the first end 411 side of the intermediate portion 413 of the first band body 410 " means being located on the first end 411 side of the first band body 410 with respect to the center of a linear distance L1 from the first end 411 to the second end 412 of the first band body 410, the center being obtained by dividing the linear distance L1 into two in the longitudinal direction of the first band body 410 in the plan view illustrated in Fig. 2.

The first band body 410 can be configured to be more opaque than the main body 110 of the cover member 100.

In the present embodiment, a part of the main body 110 of the cover member 100 is made of a transparent material so that the operator can visually recognize the marker portion 260 and the puncture site p from the outer surface 110b side of the main body 110 when placing the cover member 100 on the right hand H1 as described later. When the main body 110 is made of a transparent material as described above, the first band body 410 can be made more opaque than the main body 110, for example, by applying a predetermined color. Note that the wording "more opaque than the main body 110 " means having a smaller transmittance of visible light than the main body 110.

The color applied to the first band body 410 is, for example, white. However, the specific color applied to the first band body 410 is not particularly limited.

At least a part of the layout display portion 600 can have a color different from the color of the first band body 410. In a case where white is applied to the first band body 410 as described above, green, for example, can be applied to at least a part of the layout display portion 600. The green color has a large hue difference from red, which is the color of blood. Therefore, in a case where a green color is applied to the layout display portion 600, the layout display portion 600 is easily noticeable even if blood adheres to the first band body 410, and is easily visually recognized by the operator. However, the specific color applied to the layout display portion 600 is not particularly limited.

In Figs. 7 and 8, the colored section (white section) of the first band body 410 is clearly indicated by adding dots. Further, in the layout display portion 600 (the first graphic portion 610 and the second graphic portion 620), sections (green sections) to which a color different from that of the first band body 410 is applied are painted in black.

The colored section in the layout display portion 600 can contain, for example, a fluorescent pigment. By adding the fluorescent pigment to the layout display portion 600, in a case where the hemostatic device 10 has been removed from the hand H of the patient at night or the like, the operator can quickly notice such a state.

In addition, the specific configuration (patterns, symbols, illustrations, and the like) of the layout display portion 600 (the first graphic portion 610 and the second graphic portion 620) is not particularly limited as long as it has a function of assisting the work to place the hemostatic device 10 on the right hand H1 and the left hand H2 of the patient.

As illustrated in Figs. 7 and 8, the layout display portion 600 can be disposed only on the outer surface 410b of the first band body 410.

Since the layout display portion 600 is disposed only on the outer surface 410b of the first band body 410, the operator can clearly recognize the shape and the symbol when viewing the layout display portion 600 from the outer surface 410b side of the first band body 410 as illustrated in Fig. 7. On the other hand, since the layout display portion 600 is disposed only on the outer surface 410b of the first band body 410, the shape and symbol of the layout display portion 600 are unclear when the operator views the layout display portion 600 from the inner surface 410a side of the first band body 410 as illustrated in Fig. 8, as compared with the case where the operator views the layout display portion 600 from the outer surface 410b side. Therefore, when viewing the layout display portion 600 from the inner surface 410a side of the first band body 410, the operator cannot clearly recognize the layout display portion 600 as compared with the case of viewing the layout display portion 600 from the outer surface 410b side.

As illustrated in Fig. 7, the first graphic portion 610 includes positions of the inflatable member 210 and the layout display portion 600 in a state where the hemostatic device 10 is worn on the right hand H1.

The first graphic portion 610 includes a first part 611 colored as a background, a second part 612 attached with a symbol R indicating that the first graphic portion 610 shows a placement example for the right hand H1, a third part 613 indicating an illustration of the right hand H1 with the fingertip of the right hand H1 being directed to the second end 412 of the first band body 410, and a fourth part 614 attached with an illustration of the hemostatic device 10 showing an appropriate placement example of the cover member 100 provided with the inflatable member 210 and the layout display portion 600.

In addition, as illustrated in Fig. 7, the second graphic portion 620 includes a first part 621 colored as a background, a second part 622 attached with a symbol L indicating that the second graphic portion 620 shows a placement example for the left hand H2, a third part 623 indicating an illustration of the left hand H2 with the fingertip of the left hand H2 being directed to the second end 412 of the first band body 410, and a fourth part 624 attached with an illustration of the hemostatic device 10 showing an appropriate placement example of the cover member 100 provided with the inflatable member 210 and the layout display portion 600.

In the present embodiment, a color (for example, green) different from that of the first band body 410 can be applied to at least a part of the first parts 611 and 621 and the fourth parts 614 and 624 of the graphic portions 610 and 620.

As illustrated in Figs. 3 and 4, the layout display portion 600 can be configured to be positioned on the second end 412 side of the first band body 410 with respect to an end 415a of the slit 415 positioned on the first end 411 side of the first band body 410.

### <Support Member 300>

As illustrated in Figs. 3, 4, 5, and 6, the hemostatic device 10 has a support member 300 disposed on the cover member 100 so as to cover the inflatable member 210.

The support member 300 can be made of a plate-shaped material harder than the inflatable member 210. The support member 300 can be disposed in the clearance g of the holding portion 130.

As illustrated in Figs. 3, 4, 5, and 6, the support member 300 includes the upper end portion 301 that is located on the first band body 410 side and serves as an end of the support member 300, the lower end portion 302 serving as an end opposite to the upper end portion 301, and a pair of side surface portions 303 and 304 connecting the upper end portion 301 and the lower end portion 302.

The upper end portion 301 of the support member 300 can be placed on the fingertip side (distal side) of the finger in a state where the hemostatic device 10 is worn on the hand H of the patient. The lower end portion 302 of the support member 300 can be placed on the forearm Ar side (proximal side) in the state where the hemostatic device 10 is worn on the hand H of the patient. Note that the upper end portion 301 defines a "distal portion" of the support member 300, and the lower end portion 302 defines a "proximal portion" of the support member 300.

Each of the upper end portion 301 of the support member 300 and the lower end portion 302 of the support member 300 can be configured to include a flat portion extending substantially linearly in the plan views illustrated in Figs. 3 and 4.

As illustrated in Fig. 6, the upper surface 300b of the support member 300 is curved toward the second band body 420 and the third band body 430 (to the left and to the right in Fig. 6).

The upper surface 300b of the support member 300 can be formed to have a substantially constant radius of curvature at each section in the longitudinal direction (vertical direction in Figs. 3 and 4) of the support member 300, for example.

As illustrated in Fig. 6, the lower surface 300a of the support member 300 is curved toward the second band body 420 and the third band body 430 (to the left and to the right in Fig. 6) Therefore, the lower surface 300a of the support member 300 is curved to project in a direction away from the inflatable member 210 as illustrated in Fig. 6.

The radius of curvature of the lower surface 300a in the vicinity of the upper end portion 301 of the support member 300 can be set to be smaller than the radius of curvature of the lower surface 300a in the vicinity of the lower end portion 302 of the support member 300, for example.

The support member 300 can be configured such that the thickness of each of the side surface portions 303 and 304 connecting the upper end portion 301 and the lower end portion 302 decreases from the upper end portion 301 to the lower end portion 302. That is, the support member 300 can be configured to have a cross-sectional shape inclined such that a distance between the upper surface 300b and the lower surface 300a decreases from the upper end portion 301 to the lower end portion 302 in the cross-sectional view illustrated in Fig. 5 and the cross-sectional view illustrated in Fig. 14.

In the plan views illustrated in Figs. 3 and 4, the corners positioned at the four corners of the support member 300 can be formed in a rounded curved shape.

The hemostatic device 10 is preferably formed such that a section of the inflatable member 210 where the marker portion 260 is provided, a section of the cover member 100 (the upper surface region 131 and the lower surface region 132 of the holding portion 130) overlapping the marker portion 260, and a section of the support member 300 overlapping the marker portion 260 are transparent. In a case where each of the members 100, 210, and 300 is configured in this manner, an operator can easily visually confirm the position of the marker portion 260 provided on the inflatable member 210 and/or the first puncture site p1 through the sections formed to be transparent in the members 100, 210, and 300 when the hemostatic device 10 is worn on the right hand H1 of the patient as illustrated in Figs. 10, 11, and 13. Note that the term "transparent" includes being colored transparent, being colorless transparent, and being translucent.

When the inflatable member 210 is made of the above-described material, examples of a constituent material of the support member 300 include acrylic resin, polyvinyl chloride (particularly hard polyvinyl chloride), polyolefin such as polyethylene, polypropylene, or polybutadiene, polystyrene, poly-(4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, and polyethylene terephthalate (PET).

### <Injection Member 281>

The injection member 281 includes a connector having an incorporated check valve (not illustrated). A syringe (not illustrated) can be connected to the injection member 281. The injection member 281 is connected to the inflatable member 210 using the tube member 283.

A buffer member 282 having an inflatable space is disposed between the injection member 281 and the inflatable member 210. The buffer member 282 is constituted by a flexible bag-shaped member in which a space is formed. Note that the buffer member 282 may be provided with an arrow-shaped marker indicating an insertion direction of the syringe into the injection member 281.

The injection member 281 is connected to one end of the buffer member 282. A lumen of the injection member 281 communicates with the space of the buffer member 282. However, while the check valve incorporated in the injection member 281 is closed, communication between the lumen of the injection member 281 and the space of the buffer member 282 is blocked.

The tube member 283 having flexibility is connected to the other end of the buffer member 282. A lumen of the tube member 283 communicates with the space of the buffer member 282. Furthermore, the other end of the tube member 283, opposite to the one end connected to the buffer member 282, is connected to the inflatable member 210. The lumen of the tube member 283 communicates with the inner cavity 210a of the inflatable member 210.

To inflate the inflatable member 210, the operator inserts a distal tubular portion of the syringe (not illustrated) into the injection member 281 to open the check valve. The operator injects air in the syringe into the inner cavity 210a of the inflatable member 210 by pushing a plunger of the syringe in a state where the check valve of the injection member 281 is open.

When the air is injected into the inner cavity 210a of the inflatable member 210, the inflatable member 210 is inflated. When the inflatable member 210 is inflated, the buffer member 282 communicating with the inner cavity 210a of the inflatable member 210 by means of the tube member 283 expands. The operator can easily understand that the inflatable member 210 is inflated without leakage of air by visually confirming expansion of the buffer member 282.

To deflate the inflatable member 210, the operator inserts the distal tubular portion of the syringe into the injection member 281 and pulls the plunger of the syringe. By performing the operation described above, the operator can discharge the air in the inner cavity 210a of the inflatable member 210 to the syringe.

Note that the injection member 281, the buffer member 282, and the tube member 283 may be prepared and provided in a state of being coupled to the inflatable member 210, or may be prepared and provided in a state of being separated from the inflatable member 210. In addition, the injection member 281 is not particularly limited as to a specific configuration or the like as long as the supply of the fluid to the inner cavity 210a of the inflatable member 210 and the discharge of the fluid from the inner cavity 210a of the inflatable member 210 can be controlled.

### <Usage Example of Hemostatic Device>

Next, a usage example and operational effects of the hemostatic device 10 will be described.

Hereinafter, a first usage example (see Figs. 10 to 16) of stopping bleeding at the first puncture site p1 formed in the right hand H1 and a second usage example (see Figs. 17 to 19) of stopping bleeding at the third puncture site p3 formed in the left hand H2 will be described. Note that Figs. 10 to 13 and Figs. 17 to 19 are diagrams of the hemostatic device 10 as viewed by the eyes (eyes of the operator who is assumed to be present on the fingertip side) of the operator performing the work to place and attach the hemostatic device 10 on the hand H of the patient.

The first usage example will be described with reference to Figs. 10 to 16.

Fig. 10 illustrates a state where various procedures performed using the sheath tube 710 of the introducer 700 inserted into the first puncture site p1 are completed.

When placing the hemostatic device 10 on the right hand H1 of the patient, the operator places the inflatable member 210 and the support member 300 so as to overlap the first puncture site p1 as illustrated in Fig. 10. At this time, the operator can appropriately position the inflatable member 210 and the support member 300 at the first puncture site p1 by placing the marker portion 260 at the first puncture site p1 while visually confirming the position of the marker portion 260 provided on the inflatable member 210.

When placing the hemostatic device 10 on the right hand H1 of the patient, the operator views and checks the layout display portion 600, thereby being capable of easily recognizing that the portions of the hemostatic device 10 are appropriately placed on predetermined positions of the right hand H1 and that the hemostatic device 10 is oriented in a proper direction.

When placing the hemostatic device 10 on the right hand H1 of the patient, the operator can place the support member 300 so as to overlap at least a part of the metacarpal bone B1 of the index finger and at least a part of the metacarpal bone B2 of the thumb. When, for example, stopping bleeding at the first puncture site p1, the operator can place the support member 300 on a predetermined position P1 located on the extension of the metacarpal bone B1 and the metacarpal bone B2 as illustrated in Fig. 9. In addition, when stopping bleeding at the second puncture site p2, the operator can place the support member 300 at a predetermined position P2 located on the extension of the metacarpal bone B1 and the metacarpal bone B2 and on the forearm A side with respect to the predetermined position P1 as illustrated in Fig. 9.

An interval between the metacarpal bone B1 of the index finger and the metacarpal bone B2 of the thumb gradually decreases from the fingertip side to the forearm Ar side (see Fig. 9). The width of the support member 300 decreases from the upper end portion 301 positioned on the fingertip side to the lower end portion 302 positioned on the forearm Ar side (see Figs. 3 and 4). Therefore, the operator can position the support member 300 along at least a part of the metacarpal bone B1 of the index finger and at least a part of the metacarpal bone B2 of the thumb by placing the support member 300 so as to overlap the metacarpal bone B1 of the index finger and the metacarpal bone B2 of the thumb from the dorsal side of the right hand H1 of the patient. The operator can prevent the formation of a gap between the inflatable member 210 located on the lower surface 300a side of the support member 300 and the right hand H1 by placing the support member 300 along at least a part of the metacarpal bone B1 of the index finger and at least a part of the metacarpal bone B2 of the thumb.

As illustrated in Fig. 11, the operator wraps the second band body 420 and the third band body 430 along the outer periphery of the right hand H1 of the patient. The operator can connect the second band body 420 and the third band body 430 by means of the securing parts 510 and 530 by bringing the third securing part 530 (see Fig. 2) provided on the inner surface of the third band body 430 into contact with the first securing part 510 (see Fig. 1) provided on the outer surface of the second band body 420. The operator can firmly wrap the band bodies 420 and 430 along the outer periphery of the right hand H1 by connecting the band bodies 420 and 430 to each other in a state where the support member 300 overlaps at least a part of the metacarpal bone B1 of the index finger and at least a part of the metacarpal bone B2 of the thumb.

The upper surface 300b of the support member 300 is curved toward the second band body 420 (toward the side surface portion 303) and the third band body 430 (toward the side surface portion 304) (see Fig. 6). Therefore, the operator can press the support member 300 against the right hand H1 of the patient from the upper surface 300b side along the outer periphery of the right hand H1 as illustrated in Fig. 15 by wrapping the second band body 420 and the third band body 430 around the right hand H1. Thus, the hemostatic device 10 can increase the securing force for securing the support member 300 and the inflatable member 210 to the right hand H1 of the patient.

As illustrated in Fig. 13, the operator places a part of the first band body 410 on a palm side of the right hand H1 of the patient while passing the first band body 410 through the interdigital space fb located between the thumb and the index finger of the right hand H1 of the patient. When doing so, the operator brings the fourth securing part 540 (see Fig. 2) provided on the inner surface of the first band body 410 into contact with the second securing part 520 (see Fig. 1) provided on the outer surface of the third band body 430, thereby being capable of connecting the first band body 410 and the second band body 420 by means of the securing parts 520 and 540.

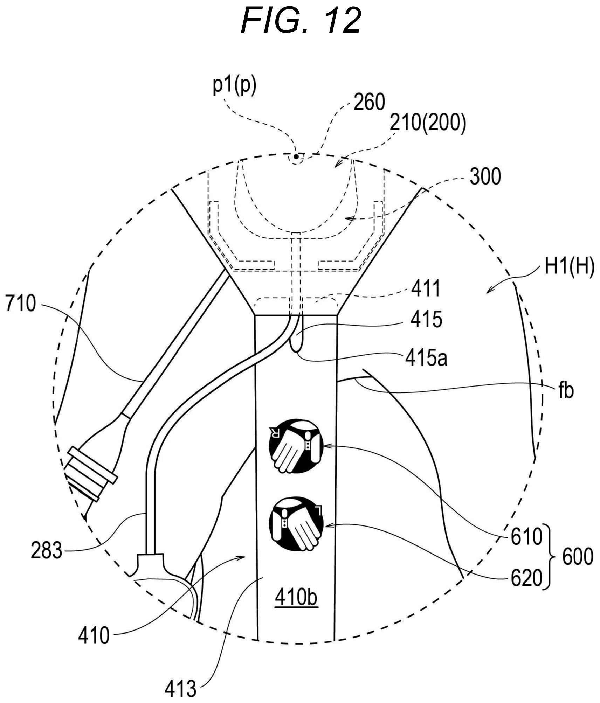

Fig. 12 is an enlarged view of the layout display portion 600 viewed by the operator when the operator places the first band body 410 in the interdigital space fb.

When placing the first band body 410 in the interdigital space fb, the operator views the layout display portion 600 and compares the content indicated by the layout display portion 600 with the state of the placement work the operator is doing, thereby being capable of ascertaining whether or not the hemostatic device 10 is placed on the right hand H1 in an appropriate orientation and posture. Specifically, as illustrated in Fig. 12, the operator can easily ascertain whether or not the hemostatic device 10 is placed in an appropriate orientation on the right hand H1 of the patient by comparing the content indicated by the first graphic portion 610 viewed by the eyes of the operator with the actual orientation of the right hand H1 of the patient, the position of the interdigital space fb, and the like.

In addition, in the hemostatic device 10, the layout display portion 600 is located on the first end 411 side of the intermediate portion 413 of the first band body 410. Therefore, when, for example, the operator holds the second end 412 of the first band body 410 located on the operator side with his/her fingers during placement of the first band body 410 in the interdigital space fb, it is possible to prevent the layout display portion 600 from being covered by the fingers. Therefore, the operator can appropriately place the hemostatic device 10 on the right hand H1 of the patient in accordance with the content indicated by the layout display portion 600.

In addition, in the hemostatic device 10, the first band body 410 is made of a material more opaque than that of the main body 110, and the layout display portion 600 has a color different from that of the first band body 410. Therefore, the operator can easily recognize the first band body 410 when viewing the first band body 410. Furthermore, the operator can easily recognize the graphic portions 610 and 620 disposed on the first band body 410 by viewing the layout display portion 600 having a color different from that of the first band body 410.

In addition, in the hemostatic device 10, the layout display portion 600 is disposed only on the outer surface 410b of the first band body 410. Therefore, when viewing the layout display portion 600, the operator confirms that the layout display portion 600 is clearly displayed on the first band body 410, so that the operator can easily ascertain whether or not the outer surface 410b and the inner surface 410a of the first band body 410 (the outer surface 110b and the inner surface 110a of the main body 110 of the cover member 100) are erroneously inverted (placed inside-out).

In addition, in the hemostatic device 10, the first graphic portion 610 includes the positions of the inflatable member 210 and the layout display portion 600 in a state where the hemostatic device 10 is worn on the right hand H1, and the second graphic portion 620 includes the positions of the inflatable member 210 and the layout display portion 600 in a state where the hemostatic device 10 is worn on the left hand H2. Therefore, when placing the first band body 410 in the interdigital space fb, the operator can easily and clearly ascertain whether or not the hemostatic device 10 is placed in an appropriate orientation by checking the placement state of the hemostatic device 10 in accordance with the content indicated by the first graphic portion 610 and the content indicated by the second graphic portion 620 of the layout display portion 600.

In addition, in the hemostatic device 10, the tube member 283 is inserted from the inner surface 410a side of the first band body 410 toward the outer surface 410b side of the first band body 410 through the slit 415. Therefore, the operator can prevent the tube member 283 from unnecessarily moving by locating a part of the tube member 283 in the slit 415 when placing the hemostatic device 10 on the right hand H1 of the patient. This improves operability when the operator handles the hemostatic device 10. In addition, in a state where the hemostatic device 10 is worn on the right hand H1 of the patient, the tube member 283 is inserted into the slit 415, and a part of the tube member 283 is drawn out and placed on the outer surface 410b side of the first band body 410, whereby the tube member 283 can be prevented from being pressed against the right hand H1 of the patient by the first band body 410 placed in the interdigital space fb. In addition, the layout display portion 600 is located on the second end 412 side of the first band body 410 with respect to the end of the slit 415 located on the first end 411 side of the first band body 410. Therefore, when placing the hemostatic device 10 on the right hand H1 of the patient, the operator can also prevent the tube member 283 from covering the layout display portion 600 while preventing the tube member 283 from unnecessarily moving due to the slit 415. This improves operability when the operator handles the hemostatic device 10.

In the hemostatic device 10, the first band body 410 is connected to the main body 110 at a position facing the curved region 133 across the support member 300 (see Figs. 3 and 4). Therefore, as illustrated in Figs. 11 and 12, when wrapping the first band body 410 around the interdigital space fb, the operator can confirm the positional relation between the inflatable member 210 and the support member 300 while viewing the layout display portion 600. Thus, the operator can easily and appropriately place the hemostatic device 10 on the right hand H1 of the patient while aligning the inflatable member 210 and the support member 300 with the first puncture site p1.

The right hand H1 of the patient has an inclined part in each section (for example, the vicinity of the snuff box Sb) (see Fig. 9). Furthermore, a shape of the inclined part of the right hand H1 depends on individual differences of patients. For example, in a case where the inner surface of the support member is formed in a flat shape, it is difficult to place the support member along the inclined part of the right hand H1. In the hemostatic device 10 according to the present embodiment, the lower surface 300a of the support member 300 is curved to project in a direction away from the inflatable member 210 (see Figs. 6 and 15). Therefore, when the lower surface 300a of the support member 300 is placed so as to overlap the inclined part of the right hand H1 of the patient, the lower surface 300a of the support member 300 can be placed along the right hand H1 of the patient. As a result, the support member 300 of the hemostatic device 10 can be placed to overlap the first puncture site p1 without depending on individual difference of the right hand H1 (or the left hand H2) of the patient or the way of placement by the operator. Thus, the support member 300 of the hemostatic device 10 can be appropriately placed at a desired position near the first puncture site p1.

Further, the lower surface 300a of the support member 300 of the hemostatic device 10 is placed along the body surface of the right hand H1 when the operator wraps the second band body 420 and the third band body 430 around the right hand H1 of the patient, whereby the band bodies 420 and 430 extending from the side surface portions 303 and 304 of the support member 300 can be placed close to or in close contact with the body surface of the right hand H1 of the patient (see Fig. 15). Thus, the hemostatic device 10 can increase the securing force for securing the support member 300 and the inflatable member 210 to the right hand H1 of the patient. As a result, even when an impact or the like is applied from the outside in a state where the hemostatic device 10 is worn on the right hand H1 of the patient, the support member 300 and the inflatable member 210 are less likely to be displaced.

In the hemostatic device 10, the layout display portion 600 is located on the first end 411 side of the intermediate portion 413 of the first band body 410. When the layout display portion 600 is disposed as described above, the distance between the first graphic portion 610 and the first end 411 of the first band body 410 is preferably set within 20 mm along the longitudinal direction of the first band body 410. In a case where the distance between the first graphic portion 610 and the first end 411 of the first band body 410 is set as described above, a part of the layout display portion 600 (a part of the first graphic portion 610) is located at a position that can be visually confirmed by the operator in a state where the hemostatic device 10 is worn on the right hand H1 of the patient as illustrated in Fig. 13. As a result, the operator can confirm that the first band body 410 is appropriately placed in the interdigital space fb by viewing the layout display portion 600 even in a stage where the placement of the hemostatic device 10 on the right hand H1 of the patient is completed.

The operator inflates the inflatable member 210 by injecting air into the inflatable member 210 in a state where the syringe is connected to the injection member 281. As illustrated in Figs. 14 and 15, when the inflatable member 210 is inflated in the hemostatic device 10, the inflatable member 210 applies a compressive force to the first puncture site p1.

When the inflatable member 210 is inflated in a state where the hemostatic device 10 is worn on the right hand H1 of the patient, the support member 300 presses the inflatable member 210 against the right hand H1 of the patient. As a result, the hemostatic device 10 can prevent the inflatable member 210 from being lifted from the right hand H1 of the patient.

The hemostatic device 10 has the constricted portion 132a in the lower surface region 132 of the holding portion 130 of the main body 110. The constricted portion 132a is configured such that a width of the lower surface region 132 decreases from the first band body 410 side to the curved region 133 side. Therefore, in a state where the hemostatic device 10 is worn on the right hand H1, the deformation of the upper surface region 131 is not inhibited by the lower surface region 132 due to the constricted portion 132a located in the lower surface region 132, and the cover member 100 can deform the upper surface region 131 along the support member 300. Therefore, in a state where the hemostatic device 10 is worn on the right hand H1, the proximal portion 122 of the cover member 100 of the hemostatic device 10 is deformed along both ends of the support member 300 in the width direction, so that a width of the proximal portion 122 of the cover member 100 can be reduced. Since the width of the proximal portion 122 of the cover member 100 decreases, the hemostatic device 10 can prevent an article or the like from being likely to be caught on the proximal portion 122 of the cover member 100. As a result, the hemostatic device 10 can improve the usability of the operator by the layout display portion 600 and improve the usability of the patient.

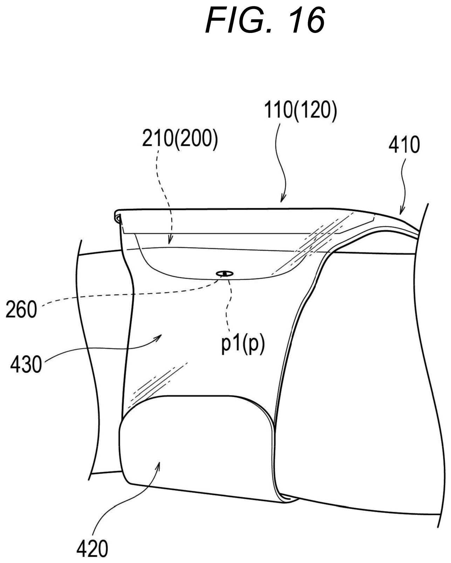

Fig. 16 illustrates the hemostatic device 10 as viewed in the direction indicated by an arrow 16A in Fig. 14 in a simplified manner in a state where the hemostatic device 10 is worn on the right hand H1 of the patient.

In the hemostatic device 10, the first band body 410 has a color more opaque than the color of the main body 110 of the cover member 100. When the first band body 410 is configured as described above, the second band body 420 and the third band body 430 extending from the main body 110 can be formed to be transparent. In a case where the second band body 420 and the third band body 430 are formed to be transparent, the operator can visually recognize the first puncture site p1 through the band bodies 420 and 430 when looking at the first puncture site p1 from the lateral side (side on which the band bodies 420 and 430 are wrapped) as illustrated in Fig. 16. Therefore, when placing the hemostatic device 10 on the right hand H1 of the patient, it is possible to easily perform alignment with respect to the first puncture site p1. In addition, in a state where the hemostatic device 10 is worn on the right hand H1 of the patient, the operator can easily check whether or not each of the band bodies 420 and 430 is excessively tightened on the right hand H1 by checking the state of the inflatable member 210 through the band bodies 420 and 430.

Note that, in the hemostatic device, it is considered that the layout display portion 600 can be disposed on the main body 110 of the cover member 100, for example. However, in a case where the layout display portion 600 is disposed on the main body 110 of the cover member 100, it is conceivable that the inflatable member 210 positioned in the main body 110 of the cover member 100 and the layout display portion 600 are positioned to overlap each other. Therefore, when the layout display portion 600 is disposed on the main body 110 of the cover member 100, it is considered that it is difficult to appropriately locate the inflatable member 210 at the first puncture site p1.

In addition, in the hemostatic device, it is also considered that, for example, the layout display portion 600 can be disposed in the vicinity of the second end of the second band body or the second end of the third band body. However, in a case where the layout display portion 600 is disposed in the vicinity of the second end of the second band body or in the vicinity of the second end of the third band body, there is a possibility that the layout display portion 600 is located at a position that cannot be visually confirmed by the eyes of the operator in a state where the band bodies are wrapped around the right hand H1 (see Figs. 11 and 13). In such a case, the operator cannot view the layout display portion 600 while performing the work to place the hemostatic device 10. In addition, in a case where the layout display portion 600 is disposed in the vicinity of the second end of the second band body or in the vicinity of the second end of the third band body, the layout display portion 600 may be hidden by the operator's fingers while the operator wraps the band bodies along the outer periphery of the right hand H1. Therefore, the operator cannot place the hemostatic device on the right hand H1 of the patient while visually confirming the layout display portion 600.

After the inflatable member 210 is inflated, the operator removes the sheath tube 710 of the introducer 700 from the first puncture site p1 as illustrated in Fig. 13. The operator confirms that there is no bleeding from the first puncture site p1 while stopping bleeding using the hemostatic device 10. In a case where there is bleeding from the first puncture site p1, the operator can adjust an injection amount of air into the inflatable member 210.

According to the above procedure, the operator can stop bleeding at the first puncture site p1 formed in the right hand H1 of the patient using the hemostatic device 10.

Next, the second usage example of the hemostatic device 10 will be described with reference to Figs. 17 to 19.

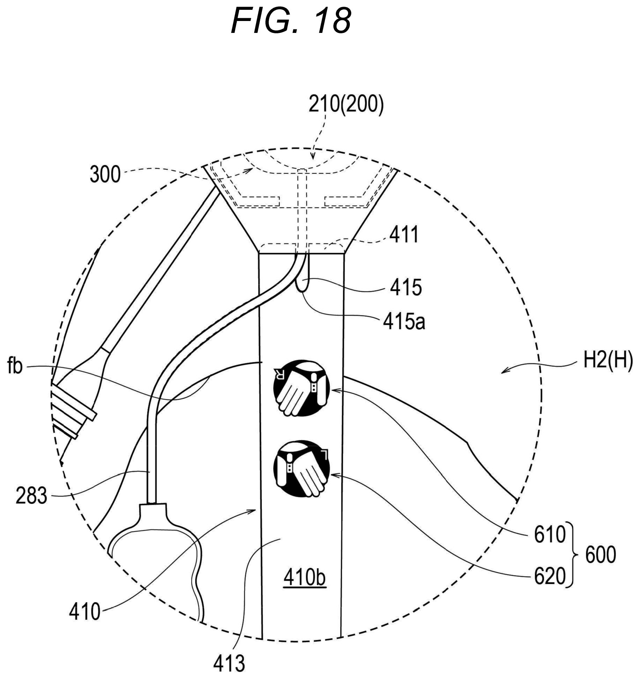

As illustrated in Figs. 17 and 18, the operator places the inflatable member 210 and the support member 300 at the third puncture site p3 formed in the left hand H2, similarly to the case where the hemostatic device 10 is placed on the right hand H1 of the patient in the first usage example. When placing the hemostatic device 10 on the left hand H2 of the patient, the operator visually confirms the layout display portion 600. The operator can easily ascertain whether or not the hemostatic device 10 is appropriately placed on the left hand H2 of the patient by visually confirming the second graphic portion 620 showing the placement example for placing the hemostatic device 10 on the left hand H2. As illustrated in Fig. 19, the operator can stop bleeding at the third puncture site p3 formed in the left hand H2 by inflating the inflatable member 210 in a state where the hemostatic device 10 is appropriately placed on the left hand H2 of the patient.

As described above, the hemostatic device 10 according to the present embodiment is placeable for both the right hand H1 and the left hand H2 of the patient according to the position of a puncture site p of the patient, and includes a cover member 100 configured to cover the puncture site p and a pressing member 200 connected to the cover member 100 and configured to compress the puncture site p, wherein the cover member 100 includes a main body 110 where the pressing member 200 is located, a first band body 410 configured to be placed between fingers of the patient and extending in a first direction from the main body 110, a second band body 420 extending in a second direction different from the first direction from the main body 110, a third band body 430 facing the second band body 420 across the pressing member 200 and extending from the main body 110 in a third direction different from the first direction and the second direction, and a layout display portion 600 showing a placement example of the hemostatic device 10 for the right hand H1 and the left hand H2 of the patient, the pressing member 200 and the layout display portion 600 are positioned on a center line C of the first band body 410, the layout display portion 600 includes a first graphic portion 610 showing a placement example of the hemostatic device 10 for the right hand H1 of the patient and a second graphic portion 620 showing a placement example of the hemostatic device 10 for the left hand H2 of the patient, and the first graphic portion 610 and the second graphic portion 620 are arranged side by side in a longitudinal direction of the first band body 410.

The hemostatic device 10 configured as described above is configured to be placeable on both the right hand H1 and the left hand H2 of the patient, whereby the operator can freely select the placement on the right hand H1 and the left hand H2 of the patient according to the puncture site p formed in the hand H of the patient. Therefore, the operator can treat both the right hand H1 and the left hand H2 of the patient with one hemostatic device 10, whereby convenience at the time of use is improved. In addition, the first band body 410 included in the cover member 100 of the hemostatic device 10 is provided with a layout display portion 600 showing a placement example of the hemostatic device 10 for the right hand H1 and the left hand H2 of the patient. The layout display portion 600 includes the first graphic portion 610 showing a placement example of the hemostatic device 10 for the right hand H1 of the patient and the second graphic portion 620 showing a placement example of the hemostatic device 10 for the left hand H2 of the patient. When placing the hemostatic device 10 on the hand H of the patient, the operator visually checks the first graphic portion 610 and the second graphic portion 620 provided on the first band body 410, thereby being capable of easily ascertaining whether or not the pressing member 200 and the band bodies 410, 420, and 430 included in the hemostatic device 10 are appropriately placed on the hand H. In addition, the first graphic portion 610 and the second graphic portion 620 are positioned on the center line C of the first band body 410 and are arranged side by side in the longitudinal direction of the first band body 410, whereby the graphic portions 610 and 620 can be provided to be noticeable in a relatively wide range in the width direction orthogonal to the longitudinal direction of the first band body 410. In addition, since the first graphic portion 610 and the second graphic portion 620 are positioned on the center line C of the first band body 410 and are arranged side by side in the longitudinal direction of the first band body 410 as described above, it is possible to prevent the first graphic portion 610 and the second graphic portion 620 from being entirely hidden outside the visual field of the operator even if the first band body 410 is twisted when the operator places the hemostatic device 10 on the hand H of the patient. Therefore, even in a case where the first band body 410 is twisted as described above, the operator can easily and appropriately place the hemostatic device 10 on the hand H of the patient in accordance with the content indicated by the layout display portion 600.

In the hemostatic device 10, the first band body 410 includes a first end 411 connected to the main body 110, a second end 412 located opposite to the first end 411, and an intermediate portion 413 extending between the first end 411 and the second end 412, and the layout display portion 600 is located on the first end 411 side of the intermediate portion 413 of the first band body 410.

As described above, in the hemostatic device 10, the layout display portion 600 is located on the first end 411 side of the intermediate portion 413 of the first band body 410. Therefore, when, for example, the operator holds the second end 412 of the first band body 410 located on the operator side with his/her fingers during placement of the first band body 410 in the interdigital space fb, it is possible to prevent the layout display portion 600 from being covered by the fingers. Therefore, the operator can appropriately place the hemostatic device 10 on the right hand H1 of the patient in accordance with the content indicated by the layout display portion 600.

In addition, in the hemostatic device 10, the first band body 410 is configured to be more opaque than the main body 110, and at least a part of the layout display portion 600 has a color different from the color of the first band body 410.

As described above, in the hemostatic device 10, the first band body 410 is made of a material more opaque than that of the main body 110, and the layout display portion 600 has a color different from that of the first band body 410. Therefore, the operator can easily recognize the first band body 410 when viewing the first band body 410. Furthermore, the operator can easily recognize the graphic portions 610 and 620 disposed on the first band body 410 by viewing the layout display portion 600 having a color different from that of the first band body 410.

In addition, in the hemostatic device 10, the first band body 410 includes an inner surface 410a on a side where the pressing member 200 is located and an outer surface 410b located on a side opposite to the inner surface 410a, and the layout display portion 600 is disposed only on the outer surface 410b.

As described above, in the hemostatic device 10, the layout display portion 600 is disposed only on the outer surface 410b of the first band body 410. Therefore, when viewing the layout display portion 600, the operator confirms that the layout display portion 600 is clearly displayed on the first band body 410, so that the operator can easily ascertain whether or not the outer surface 410b and the inner surface 410a of the first band body 410 (the outer surface 110b and the inner surface 110a of the main body 110 of the cover member 100) are erroneously inverted (placed inside-out).

In addition, in the hemostatic device 10, the pressing member 200 has an elliptical shape having a long axis in the longitudinal direction of the first band body 410, the first graphic portion 610 includes the positions of the pressing member 200 and the layout display portion 600 in a state where the hemostatic device 10 is worn on the right hand H1, and the second graphic portion 620 includes the positions of the pressing member 200 and the layout display portion 600 in a state where the hemostatic device 10 is worn on the left hand H2.

As described above, in the hemostatic device 10, the first graphic portion 610 includes the positions of the inflatable member 210 and the layout display portion 600 in a state where the hemostatic device 10 is worn on the right hand H1, and the second graphic portion 620 includes the positions of the inflatable member 210 and the layout display portion 600 in a state where the hemostatic device 10 is worn on the left hand H2. Therefore, when placing the first band body 410 in the interdigital space fb, the operator can easily ascertain whether or not the hemostatic device 10 is placed in an appropriate orientation by checking the placement state of the hemostatic device 10 in accordance with the content indicated by the first graphic portion 610 and the content indicated by the second graphic portion 620 of the layout display portion 600.

Further, in the hemostatic device 10, the pressing member 200 includes an inflatable member 210 inflatable by the injection of a fluid, the inflatable member 210 is connected to an injection member 281 for injecting the fluid into the inflatable member 210 by means of a tube member 283, the first band body 410 has a slit 415 extending in the longitudinal direction of the first band body 410, the tube member 283 is inserted from the inner surface 410a side of the first band body 410 toward the outer surface 410b side of the first band body 410 through the slit 415, and the layout display portion 600 is located on the second end 412 side of the first band body 410 with respect to the end 415a of the slit 415 located on the first end 411 side of the first band body 410.

As described above, in the hemostatic device 10, the tube member 283 is inserted from the inner surface 410a side of the first band body 410 toward the outer surface 410b side of the first band body 410 through the slit 415. Therefore, the operator can prevent the tube member 283 from unnecessarily moving by locating a part of the tube member 283 in the slit 415 when placing the hemostatic device 10 on the right hand H1 of the patient. This improves operability when the operator handles the hemostatic device 10. In addition, in a state where the hemostatic device 10 is worn on the right hand H1 of the patient, the tube member 283 is inserted into the slit 415, and a part of the tube member 283 is drawn out and placed on the outer surface 410b side of the first band body 410, whereby the tube member 283 can be prevented from being pressed against the right hand H1 of the patient by the first band body 410 placed in the interdigital space fb. As a result, the hemostatic device 10 can prevent the patient from feeling uncomfortable by the tube member 283 being pressed against the right hand H1 of the patient by the first band body 410. In addition, the layout display portion 600 is located on the second end 412 side of the first band body 410 with respect to the end 415a of the slit 415 located on the first end 411 side of the first band body 410. Therefore, when placing the hemostatic device 10 on the right hand H1 of the patient, the operator can also prevent the tube member 283 from covering the layout display portion 600 while preventing the tube member 283 from unnecessarily moving due to the slit 415. This improves operability when the operator handles the hemostatic device 10.

In addition, the hemostatic device 10 includes a support member 300 disposed on the cover member 100 so as to cover the pressing member 200, the main body 110 has a two-layer structure including a holding portion 130 that holds the support member 300, the holding portion 130 has an upper surface region 131 located on the upper surface 300b side of the support member 300, a lower surface region 132 located on the lower surface 300a side of the support member 300 and facing the upper surface region 131 across the support member 300, a curved region 133 connecting the upper surface region 131 and the lower surface region 132 on the proximal side of the support member 300, and the first band body 410 is connected to the main body 110 at a position facing the curved region 133 across the support member 300.

As described above, in the hemostatic device 10, the first band body 410 is connected to the main body 110 at a position facing the curved region 133 across the support member 300. Therefore, when wrapping the first band body 410 around the interdigital space fb, the operator can confirm the positional relation between the inflatable member 210 and the support member 300 while viewing the layout display portion 600. Thus, the operator can appropriately place the hemostatic device 10 on the right hand H1 of the patient while aligning the inflatable member 210 and the support member 300 with the first puncture site p1.

In the hemostatic device 10, the curved region 133 is formed by folding back a part of the main body 110 toward the first band body 410, the lower surface region 132 has a constricted portion 132a formed on the curved region 133 side, and the constricted portion 132a is configured in such a manner that a width of the lower surface region 132 decreases from the first band body 410 toward the curved region 133.

As described above, the hemostatic device 10 has the constricted portion 132a in the lower surface region 132 of the holding portion 130 of the main body 110. The constricted portion 132a is formed such that a width of the lower surface region 132 decreases from the first band body 410 side to the curved region 133 side. Therefore, in a state where the hemostatic device 10 is worn on the right hand H1, the deformation of the upper surface region 131 is not inhibited by the lower surface region 132 due to the constricted portion 132a located in the lower surface region 132, and the cover member 100 can deform the upper surface region 131 along the support member 300. Therefore, in a state where the hemostatic device 10 is worn on the right hand H1, the proximal portion 122 of the cover member 100 of the hemostatic device 10 is deformed along both ends of the support member 300 in the width direction, so that a width of the proximal portion 122 of the cover member 100 can be reduced. Since the width of the proximal portion 122 of the cover member 100 decreases, the hemostatic device 10 can prevent an article or the like from being likely to be caught on the proximal portion 122 of the cover member 100. As a result, the hemostatic device 10 can improve the usability of the operator by the layout display portion 600 and also improve the usability of the patient.

While the hemostatic device according to the present invention has been described above by way of the embodiment, the present invention is not limited only to the content described in the specification and can be appropriately changed on the basis of the description of the claims.

The specific shapes (planar shape and cross-sectional shape) of the inflatable member and the support member are not limited to the shapes illustrated in the embodiment.

The configuration of the pressing member that applies a compressive force to the puncture site is not limited to the inflatable member (balloon). The pressing member may include, for example, a resin material such as plastic configured to be able to apply a compressive force to the puncture site, a member made of gel or the like, an elastic material such as a sponge-like substance, an assembly of fibers such as cotton, metal, a member having a predetermined three-dimensional shape (spherical, ellipsoid, triangular pyramid, or the like), or a combination thereof as appropriate.

The present application is based on Japanese Patent Application No. 2023-137825 filed on August 28, 2023, the disclosure content of which is incorporated herein by reference in its entirety.

### Reference Signs List

10 Hemostatic device
100 Cover member
110 Main body
110a Inner surface of main body
110b Outer surface of main body
120 Sheet material
130 Holding portion
131 Upper surface region
132 Lower surface region
132a Constricted portion
133 Curved region
200 Pressing member
210 Inflatable member
260 Marker portion
281 Injection member
283 Tube member
300 Support member
300a Lower surface of support member
300b Upper surface of support member
301 Upper end portion of support member
302 Lower end portion of support member
303 Side surface portion of support member
304 Side surface portion of support member
410 First band body
410a Inner surface of first band body
410b Outer surface of first band body
411 First end of first band body
412 Second end of first band body
413 Intermediate portion of first band body
415 Slit
415a End of slit
420 Second band body
421 First end of second band body
422 Second end of second band body
423 Intermediate portion of second band body
430 Third band body
431 First end of third band body
432 Second end of third band body
433 Intermediate portion of third band body
510 First securing part
520 Second securing part
530 Third securing part
540 Fourth securing part
600 Layout display portion
610 First graphic portion
611 First part
612 Second part
613 Third part
614 Fourth part
620 Second graphic portion
621 First part
622 Second part
623 Third part
624 Fourth part
700 Introducer
g Clearance
C Center line of first band body
H Hand
H1 Right hand
H2 Left hand
Ar Forearm
fb Interdigital space
Sb Snuff box
B1 Metacarpal bone of index finger
B2 Metacarpal bone of thumb
T1 Extensor pollicis longus muscle tendon
T2 Extensor pollicis brevis muscle tendon
V1 Blood vessel (distal radial artery)
V2 Artery located at snuff box
p Puncture site
p1 First puncture site
p2 Second puncture site
p3 Third puncture site

## Claims

1. A hemostatic device that is placeable for both a right hand and a left hand of a patient according to a position of a puncture site of the patient, the hemostatic device comprising:
a cover member configured to cover the puncture site; and
a pressing member connected to the cover member and configured to compress the puncture site, wherein
the cover member includes a main body where the pressing member is located, a first band body configured to be placed between fingers of the patient and extending in a first direction from the main body, a second band body extending in a second direction different from the first direction from the main body, a third band body facing the second band body across the pressing member and extending from the main body in a third direction different from the first direction and the second direction, and a layout display portion showing a placement example of the hemostatic device for the right hand and the left hand of the patient,
the pressing member and the layout display portion are positioned on a center line of the first band body,
the layout display portion includes a first graphic portion showing a placement example of the hemostatic device for the right hand of the patient and a second graphic portion showing a placement example of the hemostatic device for the left hand of the patient, and
the first graphic portion and the second graphic portion are arranged side by side in a longitudinal direction of the first band body.

2. The hemostatic device according to claim 1, wherein
the first band body includes a first end connected to the main body, a second end located opposite to the first end, and an intermediate portion extending between the first end and the second end, and
the layout display portion is located on a side of the first end of the intermediate portion of the first band body.

3. The hemostatic device according to claim 1, wherein
the first band body is configured to be more opaque than the main body, and
at least a part of the layout display portion has a color different from a color of the first band body.

4. The hemostatic device according to claim 3, wherein
the first band body includes an inner surface on a side where the pressing member is located and an outer surface located on a side opposite to the inner surface, and
the layout display portion is disposed only on the outer surface.

5. The hemostatic device according to claim 1, wherein
the pressing member has an elliptical shape having a long axis in the longitudinal direction of the first band body,
the first graphic portion includes positions of the pressing member and the layout display portion in a state where the hemostatic device is worn on the right hand, and
the second graphic portion includes positions of the pressing member and the layout display portion in a state where the hemostatic device is worn on the left hand.

6. The hemostatic device according to claim 2, wherein
the pressing member includes an inflatable member inflatable by injection of a fluid,
the inflatable member is connected to an injection member for injecting the fluid into the inflatable member by means of a tube member,
the first band body has a slit extending in the longitudinal direction of the first band body,
the tube member is inserted from an inner surface side of the first band body toward an outer surface side of the first band body through the slit, and
the layout display portion is located on a side of the second end of the first band body with respect to an end of the slit located on a side of the first end of the first band body.

7. The hemostatic device according to claim 1 comprising a support member disposed on the cover member so as to cover the pressing member, wherein
the main body has a two-layer structure including a holding portion that holds the support member,
the holding portion has an upper surface region located on an upper surface side of the support member, a lower surface region located on a lower surface side of the support member and facing the upper surface region across the support member, and a curved region connecting the upper surface region and the lower surface region on a proximal side of the support member, and
the first band body is connected to the main body at a position facing the curved region across the support member.

8. The hemostatic device according to claim 7, wherein
the curved region is formed by folding back a part of the main body toward the first band body,
the lower surface region has a constricted portion formed on a side of the curved region, and
the constricted portion is configured in such a manner that a width of the lower surface region decreases from the first band body toward the curved region.
